# EUROPEAN PATENT SPECIFICATION

(11) **EP 2 101 961 B1**
(45) Date of publication and mention of the grant of the patent: **17.10.2018**
(21) Application number: 07858771.4
(22) Date of filing: 05.12.2007
(51) Int. Cl.: B25J 13/08, B25J 18/06, A61B 1/313, A61B 5/01, A61B 5/03, A61B 1/005, A61B 1/24

(54) **ROBOTIC ARM WITH SENSOR FOR AN ENVIRONEMENTAL PARAMETER**
ROBOTERARM MIT SENSOR FUR EINEN UMGEBUNGSPARAMETER
BRAS DE ROBOT AVEC UN CAPTEUR POUR UN PARAMETRE D'ENVIRONNEMENT

(30) Priority: 05.12.2006 GB 0624242
(43) Date of publication of application: 23.09.2009
(73) Proprietor: Oliver Crispin Robotics Limited, Filton Bristol BS34 7JN (GB)
(72) Inventor: BUCKINGHAM, Robert Oliver, Abingdon OX14 3AY (GB); GRAHAM, Andrew Crispin, Bristol BS16 5AH (GB)
(74) Representative: Fischer, Michael Maria
(86) International application number: PCT/GB2007/004645
(87) International publication number: WO 2008/068478

(56) References cited:
- EP-A- 1 319 741
- WO-A-00/79546
- WO-A-02/47755
- WO-A-97/44089
- WO-A-98/29032
- WO-A-02/100608
- WO-A-2006/136827
- DE-A1- 4 408 730
- JP-A- 6 181 882
- JP-A- 02 099 029
- JP-A- 10 024 012
- US-A- 5 386 741
- US-A- 5 933 002

## Description

This invention relates to robotic arms, and in particular to robotic arms having 'tip following' capability.

In the field of robotics there has been considerable development of robotic arms having a tip following capability; that is to say, the arm is capable of advancing longitudinally of itself in a snakelike manner. Such arms can carry a workload or tool and can be used for inspection of equipment with restricted access, such as the internal parts of a jet engine.

Tip-following devices in general are used extensively in the medical field in the form of endoscopes and to some extent in industry as boroscopes. These devices, however, rely upon the external environment to guide the work head or inspection mechanism to an appropriate location at which to perform a given task. Endoscopes are usually guided by body orifices and while a certain amount of control may be exercised at an extremity of an endoscope, the fact remains that the main body of an endoscope is essentially passive and deformable, such that the only guidance is provided by the orifice or conduit within which the device moves.

A major advance in tip following technology for robotic arms is described in our co-pending Patent Application No. WO2006/136827. This application describes and claims a robotic arm comprising a plurality of longitudinal segments, each of which comprises a plurality of passive articulated links. The link at the end of each segment is "guided" by wires so that by varying the length of the wires, the segments of the arm can be caused to bend. In a typical arm, it is possible to have a number of segments, say five or 6, each containing say 10 to 20 links. By adjusting the tension in the control wires for each segment, the arm can move and adopt various spatial shapes and configurations. This may be done for example by winding each control wire on or off a spindle using a motor. The motors are controlled for example by a computer control system.

With such an arm, the articulation of the links is generally maintained under compression, i.e. by providing tension in each of the control wires, so as to "stiffen" each articulation. This gives the arm spatial determinacy, and results in greater control over the spatial positioning of the arm. Thus it is possible to control the group of links within a segment, rather than each link individually, using the stiffness of the link design to transmit loads so that any movement and/or load change or moment change is distributed among the links in the segment. Our co-pending application WO02/100608 also relates to robotic arms where, by interposing a layer of rubber or elastomer either bonded or keyed to two members constituting the articulation between adjacent links within a segment, the rubber layer constitutes a fixed contact surface between the articulated components while at the same time providing the resilient shear capacity necessary to produce "stiffness" of the joint.

As an additional well known capability of a tip following robotic arm, the body of the arm can be moved without moving the tip of the arm. This has utility in many situations, such as using the body of the device to move an object, or moving the body to avoid moving obstacles whilst maintaining the tip of the device stationary.

However for a tip following robotic arm to conduct this type of procedure the motion controller must either be programmed with information in advance concerning where such obstacles are and how they are moving or it must be able to receive information in real time about the environment.

In many situations the former is not practical. For instance, when considering a 3D volumetric search of a car, it may not be possible to gain access to an appropriate computer model of the car, and the actual condition of the car and contents, such as driver or luggage, would necessarily make the model incorrect. It would therefore be advantageous to be able to search the car without a model of the car. Ideally environmental information would not be merely the location of objects, but also information about temperature or chemical composition or light levels or other relevant characteristics that might affect motion decisions. It would also to advantageous to search a car for chemicals, biological agents, explosives or radioactive material. It may also be advantageous to measure light levels or the temperature of the car.

As a further example, when considering operations within the human body, a model derived from an MRI scan or equivalent may not be adequate to pre-plan paths and conduct an operation with such an arm. It is known that organs move both passively, due to gravity, and actively, e.g. when conducting beating heart surgery. In such a situation it would be advantageous to be able to measure proximity to objects or varying pressure along the length of the device and move accordingly. It would also be advantageous to measure temperature and chemical composition and other such environmental variables.

In both of these examples it would be appropriate to create models of the environment from data received from the robot arm itself. Such a model could then be used to aid in the process of motion control or to assist a user. Simultaneous Localisation and Mapping (SLAM) is a current well known research topic. SLAM currently proposes use of a camera at the tip of the robot arm to provide information about the environment.

In further consideration of surgery using a tip following robotic arm, it would also be advantageous to be able to plan a route through the human body to an operation site to minimize damage to intervening organs or tissues. A critical part of this process is the ability to choose the point of access and the orientation of approach into the body. For example, when reaching up to the heart it may be appropriate to enter obliquely below the ribs near the diaphragm rather than almost perpendicular to the rib cage.

With such arms, the torsional stiffness of the arm is important. Consider an arm working in the horizontal plane, with a 90 degree curve at some point along its length. The proximal section of the arm before the bend will experience a torsional load due to self-weight of the arm and any payload or external applied forces, which will tend to twist the arm out of plane. Uncontrolled motion or deviation from desired motion is generally undesirable. Whilst it is possible to predict, calculate, or measure the uncontrolled, deformed shape and compensate for the shape deformation using the control and actuation means, it would be preferably to reduce the deformation so that its effect is reduced, ideally to a negligible level.

This invention aims to address these problems.

US 5 386 741 A relates to a robotic snake. The document reveals improvements in a flexible robotic limb which can function as a robotic snake. These improvements are particularly pertinent to miniaturization applications such as catheters or positioners for microsurgery, micro-assembly, micro-manipulation, or micro-exploration. This invention develops detailed new component designs, and it combines the basic structure with systems for sensing, control, and signal transmission. These systems include pressure sensors, length sensors, protective skins, ultrasonic imagers, cutting tools, and multiplexing schemes.

While the present invention is defined in the independent claim 1, further aspects of the invention are defined in the dependent claims, the drawings and the following description.

According to the present invention, there is provided a robotic arm arranged to advance into an environment, the arm having a controller arranged to control the shape thereof, the arm comprising at least one sensor provided along the length of the arm, the sensor being arranged to sense a parameter of the environment at or adjacent the sensor, and the controller being arranged to receive information from the sensor and use the information to control the shape of the arm.

Thus the sensor may supply information relating to the environment external to the arm at a position along the length thereof such as the presence of an object. Control of the arm can be adjusted in order to move the body of the arm towards or away from the object. The or each sensor may alternatively or in addition sense a different parameter of the environment, such as temperature, chemical composition, colour etc.

Preferably, the arm comprises a sheet material skin arranged at least partially to cover the outer surface of the arm, and the or each sensor is provided by the skin. For example, the skin may be a sensorised skin, such as a skin formed of pressure sensitive material. Alternatively the skin may comprise a grid of conductors. Thus, contact with an object in the environment can be measured by the skin, allowing the arm to move towards the object and grip the object or move away from the object and avoid the object.

Preferably sensors are distributed along the length of the arm, where any part of the arm may come in contact with an object. Thus a skin may cover the entire arm, or selected areas of the arm.

The skin may for example comprise sensor segments such as a series of rings or a series or longitudinal strips or patches, or other appropriate shapes. Skins having sensors for different parameters may also be used. Thus such an arrangement would allow for different features of the environment to be measured at various positions along the length of the arm.

Alternatively sensitivity of the skin may be modified in time to vary sensitivity or to measure different features of the environment. For example, such modifications may be made by reducing the sensitivity of a sensor element or group of elements or by not using data from some elements. For example, when moving at a high speed through an environment, it may only be necessary to detect the existence of an obstacle and hence the sensitivity of the skin may be reduced or some of the elements may be switched off. When moving more slowly, perhaps in a more confined space, then higher definition of the environment may be appropriate, such that the skin sensitivity may be increased, or more elements may be used.

It is also advantageous for the sensorised skin to be flexible. Tip following robotic arms will tend to have a long slender aspect ratio and be able to take up continuously curving shapes. The sensorised skin is thus arranged to conform to such continuously curving shapes.

Advantageously, the robotic arm is of the 'tip following' type, comprising a plurality of articulated links which are divided into segments, with one link in each segment being controllable by means of the controller. With such an arrangement, the remaining links of the segment are 'passive', essentially adopting a continuous curved shape.

In a specific embodiment of the sensorised skin, the skin may be made of a composite material such as "quantum tunnelling composite" (QTC) which is described in PCT/GB00/02402. This material is a composite of a non-conducting deformable medium such as a polymer and particles of a conductor such as a metal. It can be manufactured in thin sheet form. By choosing the ratios of conductor to non conducting material, and by choosing the material, it is possible to construct thin layers that exhibit marked change in resistance when pressure is applied or when there is a temperature change. Furthermore it is possible to select a polymer which may react with chemicals or biological agents in the environment to cause a change in resistant that can also be measured using standard electronics.

With a sensorised skin with many sensing elements it will be appreciated that there may be many signals. These signals may be transmitted remotely, for example by radio. Alternatively signals may be passed both up and down the arm (or either) in order to regulate the operation of the sensors and also to receive the data. It has been found that by applying the QTC to one or more conducting or partially conducting substrate layers the substrate may be used to provide power and ground to the sensor and also transmit signals.

Thus, the skin may comprise a material including at least two elongate conductors, the conductors extending the length of at least one segment, and being wound around the or each segment, each in opposing directions, and being attached adjacent each end of the or each segment, to increase the torsional stiffness of the arm. The conductors may thus serve to transmit sensor signals, and also to stiffen the arm. Preferably, there are a plurality of conductors wound around the or each segment in each direction at substantially evenly spaced locations around the circumference thereof. For example the skin may comprise a mesh of conductors, such as a woven mesh of wires. Each wire thus follows a helical path, preferably of about or at least about 360 degrees. The conductors are preferably stiff in tension.

With such an arrangement the minimum number of wires wrapping each segment is preferably four: two in each direction, preferably terminating diametrically opposite each other at the segment ends. In order to distribute the load and obtain a thin mesh, it is preferable to use a much larger number of wires.

Such a mesh can be extended and compressed longitudinally whilst retaining its tubular shape. Thus it allows for bending of the arm.

An example is a fabric made of conducting thread. Such fabrics are flexible and hence can conform to the surface of the arm. Some such fabrics are flexible but do not stretch, such as "Bobbinet". Such a fabric is suitable since the location of the fibres within the fabric are known. It will be appreciated that in order to measure the location of any contact with the arm it is necessary to know with appropriate accuracy where the contact was made.

Thus the skin may comprise a plurality of layers; for example a QTC layer sandwiched between two layers including conductors, as described above. Connection to a patch of skin may be by means of a fabric bus, i.e. a flexible connection. In one embodiment it may be appropriate to have the conducting fibre or fibres in different layers oriented orthogonal to each other. Thus the position of a change in resistance of the QTC can be measured by having a constant or varying input and measuring the output. The input may be a time varying input such as varying DC levels or AC or other functions.

The skin may be supported at at least one point along the segment length, so as to alleviate the tendency to buckle or cripple locally at any point along the length of a segment. This supports the skin in order to avoid crippling, which may cause the skin to lose some torsional stiffness, since the individual elongate members in the skin may no longer be in tension but deform in bending instead. Furthermore, this helps to prevent the skin from collapsing when compressed, so as to avoid wrinkles becoming traps for unwanted material or causing the arm to adhere to a surface, or become interposed between internal mechanisms of the arm itself.

The skin may comprise a plurality of layers, which may be encapsulated or separated by layers of other material, for example to seal the skin. The material of the skin may be isotropic (having the same properties in all directions) or may preferably be anisotropic (having stiffness in one or more directions which is different from the stiffness of the other directions) so as to maximise performance. The skin may also provide an external barrier for the arm which may be used to protect any internal mechanism from the environment or vice-versa. For example, a skin formed of conductors may act to reduce or prevent the ingress or egress of electromagnetic radiation.

The skin may alternatively be positioned at a smaller diameter than the outside of the arm, and/or may be built into the internal structure of the arm.

According to another aspect, the invention provides a robotic arm comprising: a plurality of articulated links arranged in segments, each segment comprising at least two links; a controller for controlling the position of one of the links in each segment so as to control the shape of the arm; and at least two elongate stiffening members each extending the length of at least one of the segments, and each being wound around the segment in opposing directions and secured at or adjacent each end of the segment.

For use in surgery, the robotic arm, such as a tip following arm, may be mounted on a highly configurable framework that enables appropriate choice of the point of access and the orientation of axis into the body. Such a framework can be designed to enable a number of tip following robotic arms to be mounted in close proximity without the arms impinging on each other. In the case of surgery, one objective of such a design is to allow the surgeon reasonable access to the patient whilst the robotic arms are in place. It may be preferable for the arm to enter the body through a natural orifice such as the mouth, to minimise the necessity for making incisions.

In order that the invention may be more readily understood, reference will now be made, by way of example, to the accompanying drawings, in which:
Figure 1 is a diagrammatic representation of a robotic arm in accordance with one embodiment of the present invention;
Figures 2a to 2d are perspective views of various embodiments of a sensorised skin for a robotic arm such as that shown in Figure 1;
Figures 3a to 3c are diagrammatic representations of various steps in the operation of the robotic arm;
Figure 4 is a diagram of a tip following robotic arm in accordance with one embodiment of the invention in use in surgery;
Figure 5 is a diagram of a tip following robotic arm in accordance with one embodiment of the invention and a mounting structure for use in surgery;
Figures 6 and 7 are further diagrams of a tip following robotic arm in use in surgery;

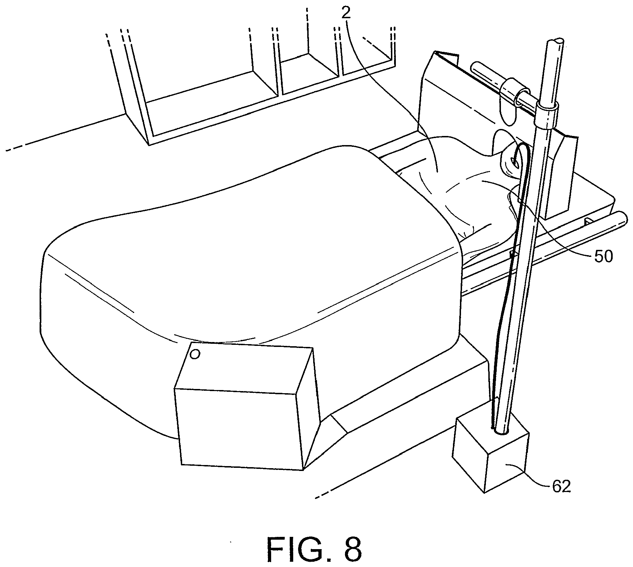

Figure 8 is a further diagram of a tip following robotic arm and mounting structure in accordance with one embodiment of the invention.
Figure 9 is a schematic perspective view of one embodiment of the tip following robotic arm,
Figure 10 is a perspective view of a segment of the arm having a skin according to one embodiment of the invention;
Figures 11A and 11B are diagrams of the construction of a multi-layer skin; and
Figures 12 and 13 are diagrams of the construction of conductors within the skin;
Figures 14A and 14B are schematic side views of a segment of a robotic arm according to an embodiment of the invention;
Figures 15A and 15B are schematic end views of the segment of Figure 14; and
Figure 16 is a schematic side view of a segment of an arm according to another embodiment of the invention.

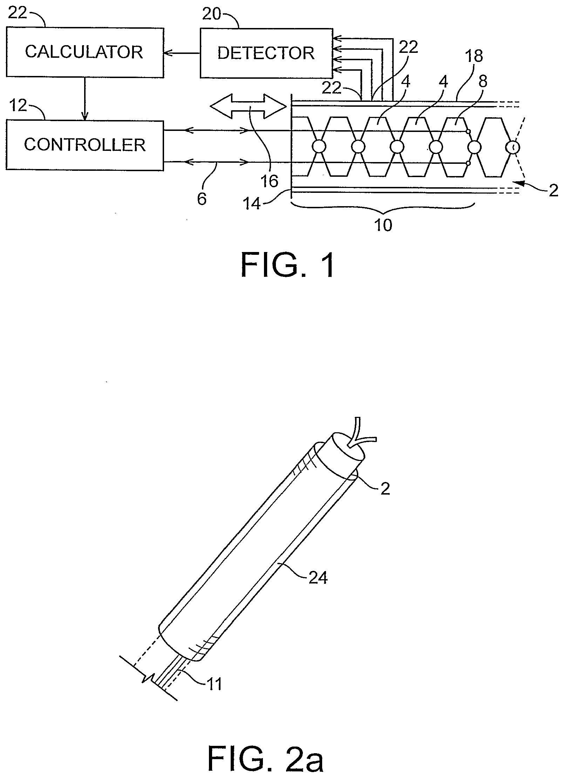

Referring to Figure 1, a robotic arm 2 comprises a plurality of articulated links 4. The position of the arm is controlled using controller 6 attached to a link 8 at the end of each segment 10. The control wires 6 are wound in or out by the controller 12 to change the shape of arm. The arm is mounted on the base plate 14 and is moveable on an introduction axis 16 so as to be able to advance or retract as the shape of the arm changes, such that the arm may follow a path in tip following snake-like manner.

The arm 2 has a tubular cover 18 which comprises a "sensorised" skin. The skin 18 may for example be touch sensitive or heat sensitive. A detector 20 measures the parameter sensed by the skin 18 at a plurality of sensor locations 22. A calculator 22 uses these measurements to calculate a modified shape of the arm 2. The information concerning the required shape is sent to the controller 12 to move the arm.

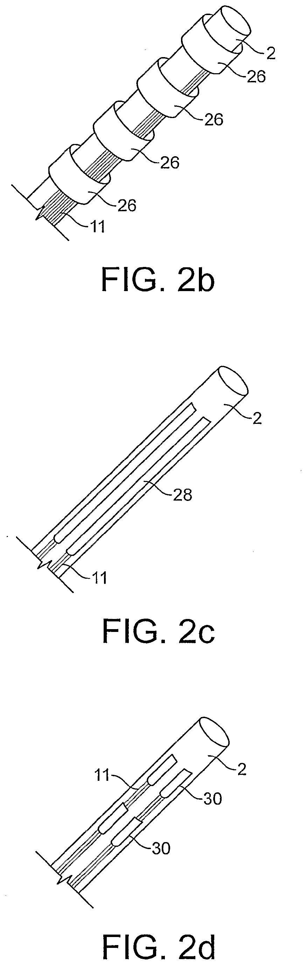

Figures 2A to 2D show various embodiments of the sensorised skin. Referring to Figure 2A, the skin may comprise a tube 24 arranged around the arm either loosely or tightly. In Figure 2B, the skin is a plurality of spaced rings 26 providing a plurality of sensors along the length of the arm. In Figure 2C the skin is provided in longitudinally arranged strips 28, which are spaced around the circumference of the arm. Figure 2D shows patches 30 of sensorised skin. In each case, the skin is connected to the detector by wires 11.

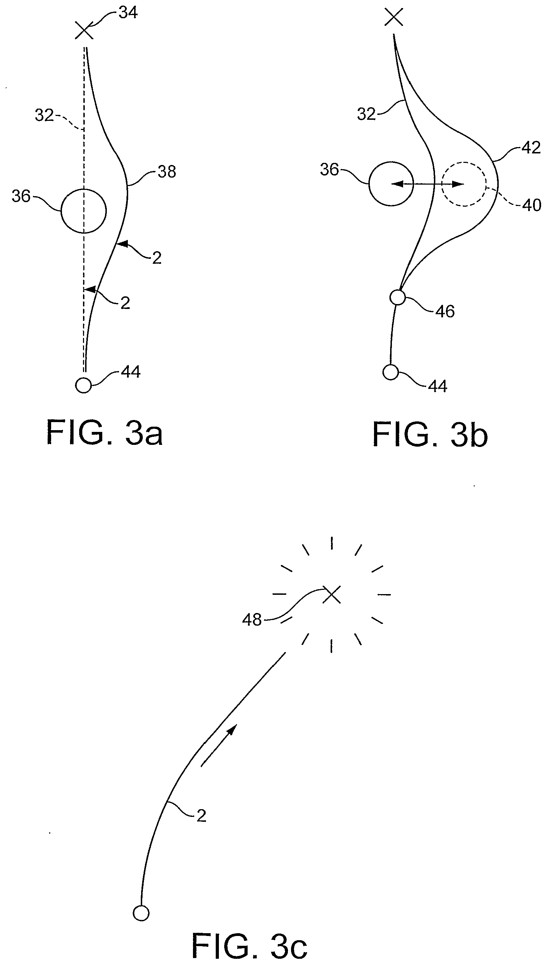

Referring to Figure 3A, an arm 2 initially advances towards a target 34 along a path 32. When the arm reaches an obstacle 36 the presence of the obstacle is detected by the sensorised skin, and a change in the path of the arm towards the target 34 is calculated and applied by the controller 12. Thus, the arm 3 adopts a new path 38 avoiding the object 36. Referring also to Figure 3B, as the object 36 moves to a new position 40, the arm may for example detect pressure from the object touching the arm. Therefore a new path 42 is calculated and applied by the controller 12. In this case, the path 42 of the arm becomes longer, such that it is necessary to advance the baseplate 14 from an initial position 44 to a new position 46.

Referring to Figure 3C it is also possible for an arm 2 to be advanced into an environment where the position of the target 48 is not known. If the sensors on the arm are able to measure a parameter of the target, such as temperature, then the arm may be drawn towards the target 48.

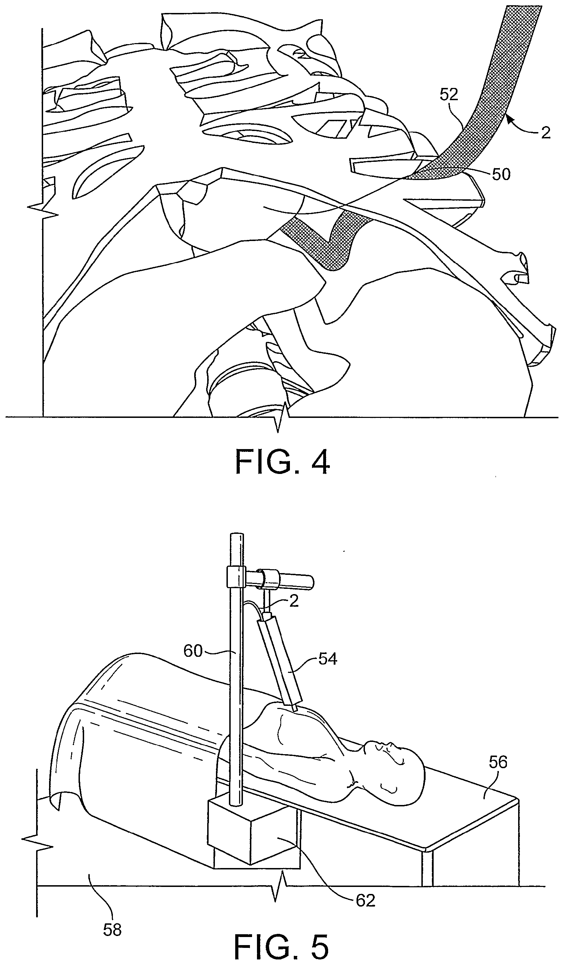

In the example of Figure 4, the robotic arm 2 is used in surgery to work on a beating heart 50. The arm 2 is covered by a sensorised skin in the form of a tube 52. The skin 52 can detect pressure caused by movement of the tissue of the heart, and the shape of arm may be adapted accordingly as the heart beats.

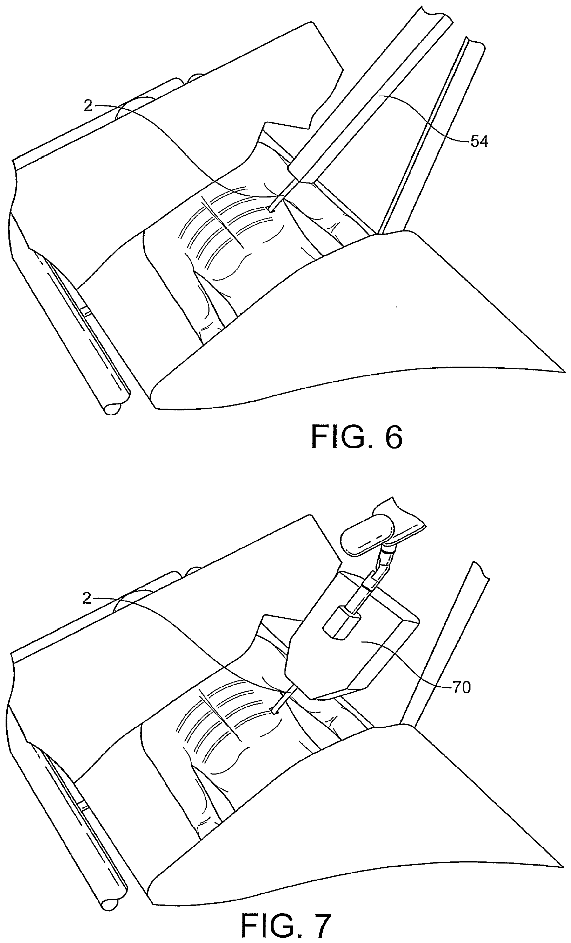

Referring to Figure 5, the robotic arm 2 used in surgery may be mounted within an arm unit 54 supported near the patient. For example, the arm unit 54 is mounted to the bed 56 or to the floor 58 on a frame 60. The frame is also attached to a control box 62 carrying the detector, calculator and controller for the arm, and the control wires for the arm 2 may run inside the frame 60. The arm 2 may be advanced or retracted by advancing or retracting the unit 54, and may also be retracted into the unit 54 when not in use (see Figure 6).

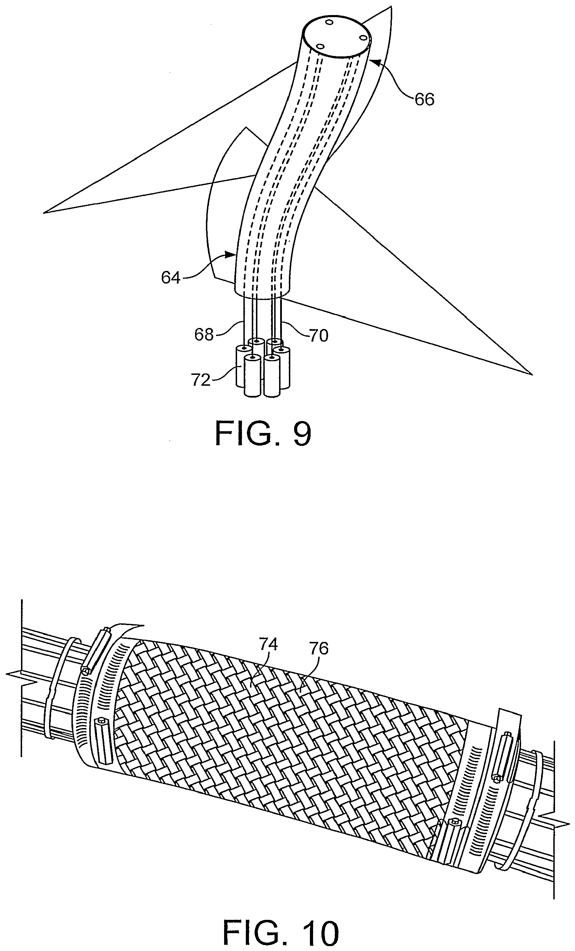

In Figure 9, two segments of a robotic arm as shown. Each segment 64, 66 is controlled by a set of three drive wires 68, 70 respectively. Each set of drive wires 68, 70 terminates at the last link in each segment 64, 66. The controller controls the position of the last link, and thus the curvature of the associated segment, by changing the length of the drive wires 68, 70 using actuators 72. Thus, the shape of the arm is determined.

Referring now to Figure 10, a skin suitable for such an arm may comprise a woven mesh of conductors 74, 76. The mesh is wound at an oblique angle to the segment, such that the conductors 74 forming the warp of the mesh weave are helically wound in one direction, and the conductors 76 forming the weft are helically wound in the opposing direction. For example, each conductor 74, 76 may be wound approximately 360 degrees around the segment. The conductors are attached to each end of segment. It will therefore be appreciated that the torsional stiffness of the segment is increased, restricting the movement of the segment substantially to bending movement and thus improving the controllability of the robotic arm.

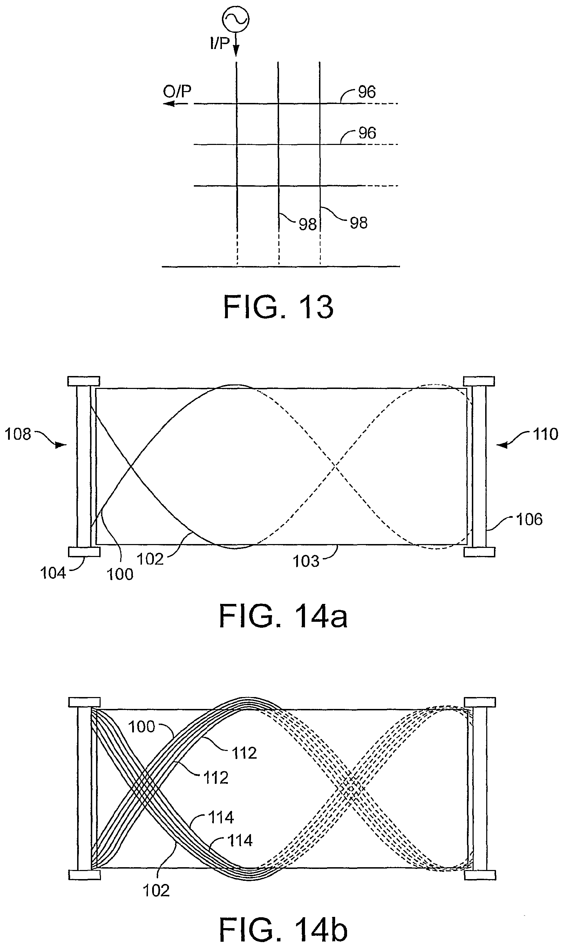

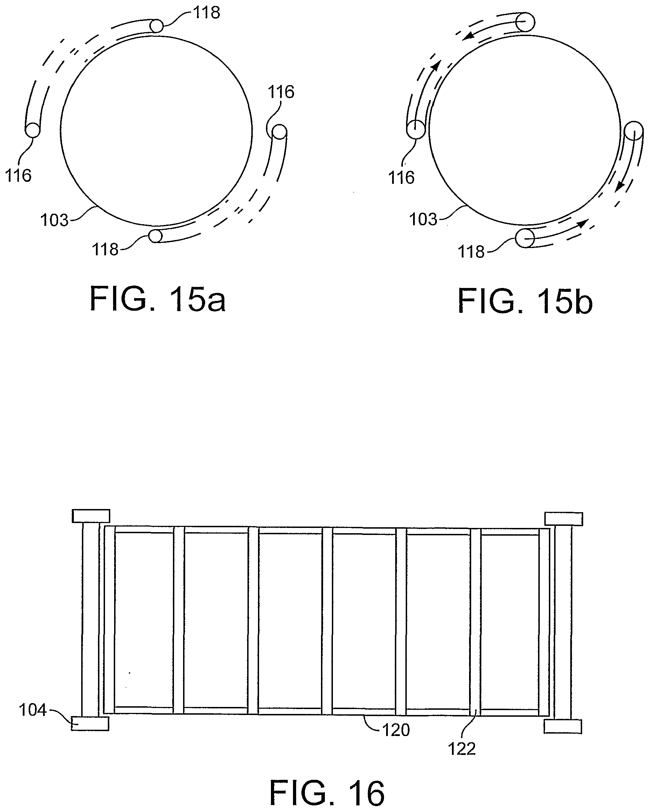

In figures 14A and 14B, further detail can be seen of how elongate members 100, 102 such as wires are wound around a segment 103, and are attached by securing bands 104, 106 to each end 108, 110 respectively of the segment 103. Further wires 112, 114 may be wound around in the same way adjacent each other to form stripes. As shown in Figure 15A and 15B, two first wires 116 are wound in a clockwise direction, and two second wires 118 are wound in an anticlockwise direction around the segment 103. The first wires 116 are evenly spaced so as to terminate at opposed positions on the circumference of the segment, the same being true of the second wires 118. As shown in Figure 16, the skin material 120 may be carrying the conductors or wires may be additionally supported between the segment ends by supports 122 to prevent creasing or crippling.

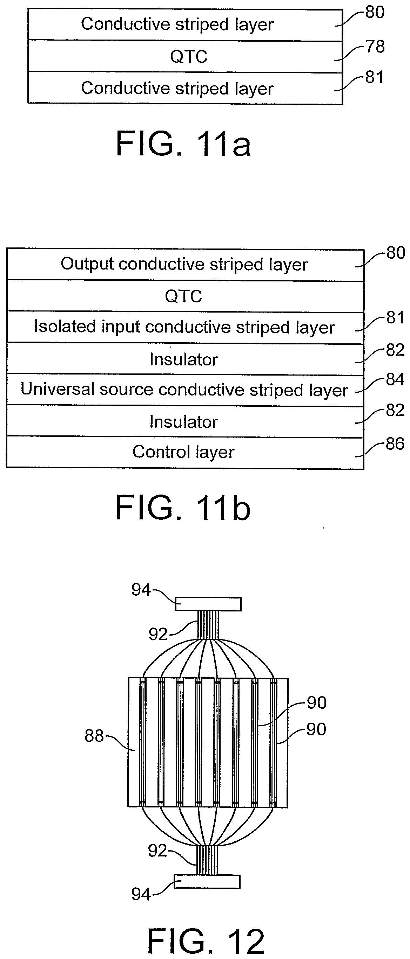

Figures 11A and 11B are examples of suitable multi-layer skins. In Figure 11A, a layer of a suitable sensorised material 78 such a QTC is sandwiched between two layers including elongate conductors; such as conductive striped layers 80, 81. In Figure 11B, further layers are added to this construction. On one side of the "sandwich" a further conductive striped layer 84, sandwiched by two insulating layers 82, is added. Finally a control layer 86 is added. With such a material the first conductive striped layer 80 is used as an output, the second conductive striped layer 81 may be an isolated input layer, and the further conductive striped layer 84 may be a universal source layer.

Figure 12 shows how the conductors and striped layers may be connected. A fabric layer 88 includes elongate conductors 90 formed as stripes. These are connected at each end via fabric buses 92 to bus terminators 94.

As shown in Figure 13, where the conductive fibres 96, 98 are oriented orthogonal to each other, the first set 98 of conductors may be used as input conductors have a varying input (for example AC), and the second set 96 of conductors may be used to measure output.

## Claims

1. A robotic arm arranged to advance into an environment, the arm having a controller arranged to control the shape thereof, the arm comprising at least one sensor provided along the length of the arm, the sensor being arranged to sense a parameter of the environment at or adjacent the sensor, and the controller being arranged to receive information from the sensor and use the information to control the shape of the arm, the robotic arm comprising a skin arranged at least partially to cover the outer surface of the arm, the or each sensor being provided by the skin, **characterized in that**
the skin comprises at least two elongate members which are stiff in tension, the members extending the length of at least one segment, and being wound around the or each segment each in opposing directions, and being attached at or adjacent each end of the or each segment, to increase the torsional stiffness of the arm, and in which the members are conductors, for transmitting sensor signals.

2. A robotic arm as claimed in claim 1 in which a plurality of sensors are distributed along the length of the arm and/or around the circumference of the arm.

3. A robotic arm as claimed in claim 1 or claim 2, comprising a plurality of sensors arranged to sense different parameters of the environment.

4. A robotic arm as claimed in claim 1, in which the skin is a flexible sheet material.

5. A robotic arm as claimed in claim 1, in which the skin is formed of pressure sensitive material.

6. A robotic arm as claimed in any of claims 1 to 5, in which the skin comprises a grid of conductors.

7. A robotic arm according to claims 1 to 6, in which the skin comprises a plurality of skin segments.

8. A robotic arm according to claims 1 to 7, in which the skin is formed of a plurality of layers of sheet material.

9. A robotic arm according to claim 1, comprising a plurality of elongate members wound around the or each segment in each direction at substantially evenly spaced locations around the circumference thereof, or a mesh of conductors, such as a woven mesh of wires.

10. A robotic arm according to claim 1, in which each member follows a helical path, preferably about or at least about 360 degrees, and at least four members wrap each segment, two in each direction, preferably terminating diametrically opposite each other at the segment ends.

11. A robotic arm according to claim 1, in which the skin comprises a Bobbinet weave fabric.

## Patentansprüche

1. Roboterarm, der so eingerichtet ist, dass er in eine Umgebung vordringen kann, wobei der Arm eine Steuerung aufweist, die dafür eingerichtet ist, seine Form zu steuern, wobei der Arm mindestens einen Sensor umfasst, der entlang der Länge des Arms bereitgestellt ist, wobei der Sensor so eingerichtet ist, dass er einen Parameter der Umgebung an oder neben dem Sensor erfassen kann, und wobei die Steuerung dafür eingerichtet ist, Informationen von dem Sensor zu empfangen und die Informationen zur Steuerung der Form des Arms zu verwenden, wobei der Roboterarm eine Haut umfasst, die mindestens teilweise eingerichtet ist, die Außenfläche des Arms zu bedecken, wobei der oder jeder Sensor von der Haut bereitgestellt wird, **dadurch gekennzeichnet, dass**
die Haut mindestens zwei längliche Elemente umfasst, die unter Zug steif sind, wobei sich die Elemente über die Länge mindestens eines Segments erstrecken und jeweils in entgegengesetzten Richtungen um das oder jedes Segment gewickelt sind und an oder neben jedem Ende des oder jedes Segments angebracht sind, um die Verwindungssteifigkeit des Arms zu erhöhen, und wobei die Elemente Leiter zur Übertragung von Sensorsignalen sind.

2. Roboterarm nach Anspruch 1, bei dem eine Vielzahl von Sensoren über die Länge des Arms und/oder über den Umfang des Arms verteilt sind.

3. Roboterarm nach Anspruch 1 oder Anspruch 2, der eine Vielzahl von Sensoren umfasst, die so eingerichtet sind, dass sie verschiedene Parameter der Umgebung erfassen.

4. Roboterarm nach Anspruch 1, bei dem die Haut ein flexibles Folienmaterial ist.

5. Roboterarm nach Anspruch 1, bei dem die Haut aus druckempfindlichem Material gebildet ist.

6. Roboterarm nach einem der Ansprüche 1 bis 5, bei dem die Haut ein Netz von Leitern umfasst.

7. Roboterarm nach Ansprüche 1 bis 6, bei dem die Haut eine Vielzahl von Hautsegmenten umfasst.

8. Roboterarm nach Ansprüche 1 bis 7, bei dem die Haut aus einer Vielzahl von Schichten von Folienmaterial gebildet ist.

9. Roboterarm nach Anspruch 1, der eine Vielzahl von länglichen Elementen umfasst, die um das oder jedes Segment in jeder Richtung an im Wesentlichen gleichmäßig beabstandeten Stellen um dessen Umfang gewickelt sind, oder ein Gewebe von Leitern, wie ein gewebtes Gewebe von Drähten.

10. Roboterarm nach Anspruch 1, bei dem jedes Element einer schraubenförmigen Bahn folgt, vorzugsweise um etwa oder mindestens um etwa 360 Grad, und mindestens vier Elemente jedes Segment umwickeln, wobei zwei in jeder Richtung, vorzugsweise diametral gegenüberliegend, an den Segmentenden enden.

11. Roboterarm nach Anspruch 1, bei dem die Haut einen Bobinet-Webstoff umfasst.

## Revendications

1. Bras de robot agencé pour avancer dans un environnement, le bras ayant un dispositif de commande agencé pour commander sa forme, le bras comprenant au moins un capteur disposé sur la longueur du bras, le capteur étant agencé pour détecter un paramètre de l'environnement sur le capteur ou adjacent à celui-ci, et le dispositif de commande étant agencé pour recevoir des informations du capteur et utiliser les informations pour commander la forme du bras, le bras de robot comprenant une peau agencée pour recouvrir au moins en partie la surface externe du bras, le ou chaque capteur étant fourni par la peau, **caractérisé en ce que**
la peau comprend deux éléments allongés qui sont rigides sous tension, les éléments étendant la longueur d'au moins un segment et étant enroulés autour du ou de chaque segment chacun dans des sens opposés et étant fixés sur chaque extrémité du ou de chaque segment ou adjacent à celle-ci pour augmenter la rigidité à la torsion du bras, et dans lequel les éléments sont des conducteurs pour transmettre des signaux du capteur.

2. Bras de robot selon la revendication 1, dans lequel une pluralité de capteurs est distribuée sur la longueur du bras et/ou autour de la circonférence du bras.

3. Bras de robot selon la revendication 1 ou la revendication 2, comprenant une pluralité de capteurs agencés pour détecter différents paramètres de l'environnement.

4. Bras de robot selon la revendication 1, dans lequel la peau est un matériau en feuille souple.

5. Bras de robot selon la revendication 1, dans lequel la peau est formée d'un matériau sensible à la pression.

6. Bras de robot selon l'une quelconque des revendications 1 à 5, dans lequel la peau comprend une grille de conducteurs.

7. Bras de robot selon les revendications 1 à 6, dans lequel la peau comprend une pluralité de segments de peau.

8. Bras de robot selon les revendications 1 à 7, dans lequel la peau est formée d'une pluralité de couches de matériau en feuille.

9. Bras de robot selon la revendication 1, comprenant une pluralité d'éléments allongés enroulés autour du ou de chaque segment dans chaque sens à des emplacements espacés de manière sensiblement régulière autour de la circonférence ou un treillis de conducteurs, tel qu'un treillis tissé de fils métalliques.

10. Bras de robot selon la revendication 1, dans lequel chaque élément suit un trajet hélicoïdal, de préférence sur environ ou au moins environ 360 degrés, et au moins quatre éléments s'enroulent sur chaque segment, deux dans chaque sens, se terminant de préférence diamétralement opposés l'un à l'autre aux extrémités des segments.

11. Bras de robot selon la revendication 1, dans lequel la peau comprend un tissu à armure bobinée.
